# EUROPÄISCHE PATENTSCHRIFT

(11) **EP 0 989 122 B1**
(45) Veröffentlichungstag und Bekanntmachung des Hinweises auf die Patenterteilung: **21.05.2003**
(21) Anmeldenummer: 99116583.8
(22) Anmeldetag: 25.08.1999
(51) Int. Cl.: C07D 233/74

(54) **Verfahren zur Herstellung von Imidazolidin-2,4-dionen**
Process for the preparation of imidazolidine-2,4-diones
Procédé de préparation d'imidazolidine-2,4-diones

(30) Priorität: 22.09.1998 DE 19843299
(43) Veröffentlichungstag der Anmeldung: 29.03.2000
(73) Patentinhaber: Degussa AG, 40474 Düsseldorf (DE)
(72) Erfinder: Beller, Matthias, Prof. Dr., 18119 Rostock (DE); Eckert, Markus, 80805 München (DE); Moradi, Wahed, 80939 München (DE)

(56) Entgegenhaltungen:
- FISHER ET AL.: J. AMER. CHEM. SOC., Bd. 64, 1942, Seiten 1434-1436, XP002126831
- W. THEILHEIMER'S: SYNTHETIC METHODS OF ORGANIC CHEMISTRY, Bd. 37, 1983, Seite 154 XP002126832 S. KARGER BASEL
- W. THEILHEIMER'S: SYNTHETIC METHODS OF ORGANIC CHEMISTRY, Bd. 42, 1988, Seite 121 XP002126833 S. KARGER BASEL
- CHEMICAL ABSTRACTS, vol. 131, no. 8, 23. August 1999 (1999-08-23) Columbus, Ohio, US; abstract no. 102515, BELLER, MATTHIAS ET AL: "Palladium-catalyzed reactions for the synthesis of fine chemicals, part 10. palladium-catalyzed synthesis of substituted hydantoins-a new carbonylation reaction for the synthesis of amino acid derivatives" XP002126836 & ANGEW. CHEM., INT. ED. (1999), 38(10), 1454-1457 ,
- "The Merck Index, 12th Ed." 1996 , MERCK & CO., INC. XP002126834 * Organic name reaction N° 58 *
- "Römpp Lexikon Chemie" 1997 , GEORG THIEME VERLAG XP002126835 * Hydantoine, page 1822 *

## Beschreibung

Die vorliegende Erfindung bezieht sich auf ein Verfahren zur Herstellung von Verbindungen der allgemeinen Formel (I) 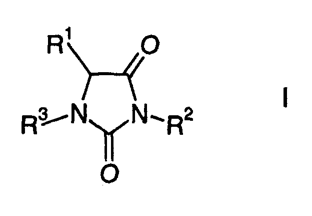 worin
R¹ bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl- (C₃-C₈)-Cycloalkyl, (C₃-C₈)-Cycloalkyl- (C₁-C₈)-Alkyl,
   wobei die oben genannten Reste einfach oder mehrfach mit Heteroatomen wie Hal, NR¹R², PO₀₋₃R¹R², OPO₀₋₃R¹R², OR¹, SR¹, SOR¹, SO₂R¹, SO₃R¹ substituiert sein können oder Gruppen wie CO₂H, CO₂R¹, CONH₂, CONHR¹ bzw. eine oder mehrere CH₂-Gruppen durch Heteroatome wie NR¹, PR¹, O oder S substituiert sein können,
R² bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl- (C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
   wobei die oben genannten Reste einfach oder mehrfach mit Heteroatomen wie Hal, NR¹R², PO₀₋₃R¹R², OPO₀₋₃R¹R², OR¹, SR¹, SOR¹, SO₂R¹, SO₃R¹ substituiert sein können bzw. eine oder mehrere CH₂-Gruppen durch Heteroatome wie NR¹, PR¹, O oder S substituiert sein können,
R³ bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
   wobei die oben genannten Reste einfach oder mehrfach mit Heteroatomen wie Hal, NR¹R², PO₀₋₃R¹R², OPO₀₋₃R¹R², OR¹, SR¹, SOR¹, SO₂R¹, SO₃R¹ substituiert sein können bzw. eine oder mehrere CH₂-Gruppen durch Heteroatome wie NR¹, PR¹, O oder S substituiert sein können.

Verbindungen der allgemeinen Formel (I) sind wichtige Ausgangsstoffe für die Synthese racemischer und enantiomerenangereicherter α-Aminosäuren, welche wiederum zur Synthese bioaktiver Wirkstoffe oder zur Ernährung von Tier und Mensch benötigt werden.

In 1- und 3-Stellung unsubstituierte Imidazolidin-2,4-dione können über die sogenannte Bucherer-Bergs Reaktion aus einem Aldehyd RCHO, HCN und Ammoniumcarbonat erhalten werden. Diese Reaktion wird zwar technisch angewandt, jedoch hat sie den Nachteil, daß die äußerst toxische Blausäure als Ausgangsmaterial eingesetzt werden muß und daß in 3-Stellung substituierte Imidazolidin-2,4-dione nicht direkt zugänglich sind.

Imidazolidin-2,4-dione, die neben der 5-Position noch in 3-Stellung einen Substituenten tragen, werden daher vorteilhafterweise aus der entsprechenden Aminosäure und einem Isocyanat dargestellt. Dieses Verfahren hat den Nachteil, daß vergleichsweise teure Aminosäuren als Edukte eingesetzt werden müssen, und daß nur wenige Isocyanate im technischen Maßstab kommerziell verfügbar sind.

1,3,5-substituierte Hydantoine können aus der entsprechenden sehr teuren N-substituierten Aminosäure und einem Isocyanat hergestellt werden (Advances in Heterocyclic Chemistry, Vol. 38, 1985, 177-228). Auch dieses Verfahren besitzt somit die oben geschilderten Nachteile. Im Falle nicht-natürlicher (nicht proteinogener) Aminosäuren muß zusätzlich die entsprechende Aminosäure vorab in mehreren Stufen hergestellt werden. Es gibt somit bis dato kein praktikables Einstufenverfahren, das die Darstellung von 1,3,5-substituierten Hydantoinen aus kostengünstigen Ausgangsverbindungen gestattet.

Aufgabe der vorliegenden Erfindung war deshalb die Angabe eines Verfahren zur Herstellung von Verbindungen der allgemeinen Formel (I), welches die oben genannten für die Verfahren des Standes der Technik geltenden Nachteile nicht innehat, insbesondere die Synthese von Verbindungen der allgemeinen Formel (I) ausgehend von preiswerten, technisch weniger risikoreichen Edukten in wenigen Stufen und mit guter Ausbeute im industriellen Maßstab gestattet.

Diese Aufgabe wird gelöst durch ein Verfahren mit den Merkmalen des kennzeichnenden Teils des Anspruchs 1. Die auf Anspruch 1 rückbezogenen Unteransprüche stellen dahingegen besondere Ausführungsformen unter Schutz.

Dadurch, daß Verbindungen der allgemeinen Formel (I) 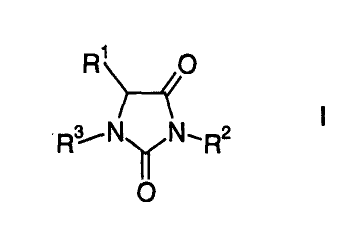 worin
R¹ bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
   wobei die oben genannten Reste einfach oder mehrfach mit Heteroatomen wie Hal, NR¹R², PO₀₋₃R¹R², OPO₀₋₃R¹R², OR¹, SR¹ , SOR¹, SO₂R¹, SO₃R¹ substituiert sein können oder Gruppen wie CO₂H, CO₂R¹, CONH₂, CONHR¹ bzw. eine oder mehrere CH₂-Gruppen durch Heteroatome wie NR¹, PR¹, O oder S substituiert sein können,
R² bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl- (C₃-C₁₉)-Heteroalkyl, (C₃-C₈)-Cycloalkyl, (C₁-C₈)-Alkyl- (C₃-C₈)-Cycloalkyl, (C₃-C₈)-Cycloalkyl- (C₁-C₈)-Alkyl,
   wobei die oben genannten Reste einfach oder mehrfach mit Heteroatomen wie Hal, NR¹R², PO₀₋₃R¹R², OPO₀₋₃R¹R², OR¹, SR¹ , SOR¹, SO₂R¹, SO₃R¹ substituiert sein können bzw. eine oder mehrere CH₂-Gruppen durch Heteroatome wie NR¹, PR¹, O oder S substituiert sein können,
R³ bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
   wobei die oben genannten Reste einfach oder mehrfach mit Heteroatomen wie Hal, NR¹R², PO₀₋₃R¹R², OPO₀₋₃R¹R², OR¹, SR¹ , SOR¹, SO₂R¹, SO₃R¹ substituiert sein können bzw. eine oder mehrere CH₂-Gruppen durch Heteroatome wie NR¹, PR¹, O oder S substituiert sein können,
dergestalt hergestellt werden, daß eine Verbindung der allgemeinen Formel (II) 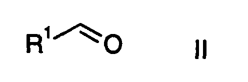 worin R¹ die oben angegebene Bedeutung annehmen kann, mit einer Verbindung der allgemeinen Formel (III) 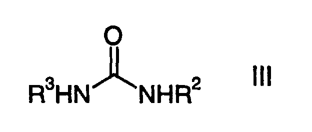 worin R² und R³ die oben angegebenen Bedeutungen annehmen können, in Gegenwart von CO und einer Pd-(0)-Verbindung oder Co-(0)-Verbindung oder Ir-(+1)-Verbindung oder Ir-(+3)-Verbindung oder Mn-(0)-, Mn-(+2)-, Mn-(+3)-Verbindung umgesetzt wird, gelangt man ausgehend von preiswerten Edukten in einem technisch einfach durchzuführenden Einstufenverfahren unter Vermeidung des Einsatzes der äußerst toxischen Blausäure in guten bis sehr guten Ausbeuten zu den gewünschten Derivaten.

Bevorzugt ist die Herstellung von Verbindungen der allgemeinen Formel (I), worin,
R¹ einen Rest ausgewählt aus der Gruppe Methyl, Ethyl, Isopropyl, n-Butyl, sec-Butyl, Isobutyl, tert-Butyl, Methylthioethyl, Thiomethyl, Methoxycarbonylmethyl, Methoxycarbonylethyl, Phenyl, 2-, 3-, 4-Pyridyl, Benzyl, 1-, 2-Phenylethyl, Hydroxymethyl, Hydroxyethyl, Vinyl, Methoxymethyl, Methoxyethyl, Carboxymethyl, Carboxyethyl, Acetamidomethyl, Acetamidoethyl, Chlormethyl, Chlorethyl, Methylphosphonoethyl, 2-Ethylhexyl, Tetradecyl, Hexadecyl bedeutet und R² und R³ unabhängig voneinander Wasserstoff, Methyl, Ethyl, Butyl, Phenyl, Benzyl, 2-Ethylhexyl bedeuten können.

Erfindungsgemäß können als Edukte beliebige Aldehyde R¹CHO, worin R¹ die vorstehend angegebene Definition hat, verwendet werden. Beispiele für geeignete Aldehyde R¹CHO sind Formaldehyd, Acetaldehyd, Propionaldehyd, Butyraldehyd, Valeraldehyd, 2-Ethylhexanal, 2-Ethylhexenal, Isobutyraldehyd, Furfural, Crotonaldehyd, Acrolein, Benzaldehyd, substituierte Benzaldehyde, Phenylacetaldehyd, 2,4-Dihydroxyphenylacetaldehyd, Glyoxalsäure, Methoxyacetaldehyd, Chloracetaldehyd, 3-Thiopropionaldehyd und α-Acetoxypropionaldehyd. Es können auch Dialdehydverbindungen und Trialdehydverbindungen eingesetzt werden. Ebenfalls geeignet sind als Ausgangsmaterialien Substanzen, die unter den.genannten Reaktionsbedingungen einen Aldehyd bilden können, z.B. Aldehydoligomere, wie Paraformaldehyd, Acetale wie Acetaldehyddimethylacetal, Allylalkohole und Epoxide wie Phenyloxiran.

Für das erfindungsgemäße Verfahren können beliebige Harnstoffe eingesetzt werden. Beispiele für geeignete Harnstoffe sind Harnstoff, Thioharnstoff, Selenoharnstoff, N-Methylharnstoff, N,N'-Dimethylharnstoff, N-Methylthioharnstoff, N,N'-Dimethylthioharnstoff, N-Benzylharnstoff, N-Phenylharnstoff, N,N'-Diphenylharnstoff, N-Phenylthioharnstoff, N-Ethylharnstoff, N-Methyl-, N'-Phenylharnstoff. Bei Verwendung von Harnstoff und monosubstituierten Harnstoffen können neben den gewünschten Hydantoinen auch die nichtcyclisierten Carbamoylaminosäuren entstehen. Diese Nebenprodukte können jedoch durch längere Reaktionszeit oder Zusatz eines wasserentziehenden Mittels cyclisiert werden, so daß lediglich die gewünschten Hydantoine gebildet werden. Falls die Synthese von Carbamoylaminosäuren oder Carbamoylaminosäureestern gewünscht ist, können diese durch Hydrolyse der Hydantoine in Gegenwart von Wasser oder Alkoholen oder enzymatisch hergestellt werden.

Die Umsetzung kann vorteilhafterweise in Gegenwart einer Säure mit einem pKs-Wert von < 5 durchgeführt werden. Als Säure können alle dem Fachman für diesen Zweck in Frage kommenden organischen oder anorganischen Säuren, wie beispielsweise HCl, HBr, Schwefelsäure, Phosphorsäure, Trifluoressigsäure, Trifluormethylsulfonsäure, p-Toluolsulfonsäure, Methansulfonsäure, Ameisensäure, Oxalsäure, HI, Benzoesäure, Essigsäure oder ein Gemisch derselben verwendet werden.

Besonders bevorzugt ist der Einsatz von Schwefelsäure oder einem Hydrogenhalogenid, wie HCl, HI oder HBr, für diesen Zweck.

Besonders bevorzugt sind Metallkatalysatoren aus Palladium- bzw. Cobalt-Verbindungen.

Als Cobaltkatalysatoren bzw. -präkatalysatoren werden vorzugsweise Cobaltcarbonyle, z. B. festes Co₂(CO)₈, eingesetzt. Das Cobaltcarbonyl kann jedoch auch aus bekannten Cobalt(II)- und Cobalt(III)-verbindungen, wie z. B. Cobalt(II)acetat, Cobalt(II)chlorid, Cobalt(II)bromid in Gegenwart von CO, ggf. unter Zusatz von H₂, in situ gebildet werden.

Als Palladium-präkatalysatoren können beliebige Palladium(II)-verbindungen, Palladium(0)-verbindungen und Palladium auf Trägermaterialien, wie beispielsweise auf Palladium auf Aktivkohle, eingesetzt werden. Beispiele für Palladium(II)-verbindungen sind Palladiumacetat, Palladiumhalogenide, Palladiumnitrile, Palladiumnitrate, Palladiumcarbonate, Palladiumketonate, Palladiumacetylacetonate sowie Allylpalladiumkomplexe. Besonders bevorzugte Vertreter sind PdBr₂, PdCl₂, Li₂PdBr₄, Li₂PdCl₄ und Pd(OAc)₂. Beispiele für Palladium(0)-verbindungen sind Palladiumphosphinkomplexe und Palladiumolefinkomplexe. Besonders bevorzugte Vertreter sind Palladium(dba)-komplexe (dba = dibenzylidenaceton) und Pd(PPh₃)₄.

Beim Einsatz von Palladiumphosphinkomplexen haben sich zudem besonders Bisphosphin-Palladium(II)-Verbindungen bewährt. Die Komplexe können als solche eingesetzt werden oder in der Reaktionsmischung aus einer Palladium-IIverbindung, wie z. B. PdBr₂, PdCl₂ oder Palladium-II-acetat unter Zusatz von Phosphinen, wie z. B. Triphenylphosphin, Tritolylphosphin, Bis-(diphenylphosphino)-ethan, 1,4-Bis-(diphenylphosphino)-butan oder 1,3-Bis-(diphenylphosphino)-propan, erzeugt werden.

Von den genannten Palladiumphosphinkomplexen sind Bistriphenylphosphin-Palladium(II)bromid-PdBr₂(PPh₃)₂- und das entsprechende Chlorid besonders bevorzugt. Diese Komplexe können als solche eingesetzt werden oder in der Reaktionsmischung aus Palladium(II)bromid bzw. -chlorid und Triphenylphosphin erzeugt werden.

Weiterhin besonders bevorzugt ist der Einsatz von Ir-(+1)-Verbindungen wie [Ir(COD)Cl]₂, [Ir(COD)₂]⁺BF₄⁻, Ph₃PIrCl(CO), Ir(CO)₂acac, Ph₃PIrH(CO) und Ir-(+3)-Verbindungen, wie IrCl₃, IrBr₃, Ir(acac)₃, Ir₄(CO)₁₂ oder Ir(OAc)₃. Daneben sind auch Mn-Katalysatoren in den Oxidationsstufen 0, +2, +3 wie Mn₂(CO)₁₀, MnBr₂, Mn(OAc)₃ als Katalysatoren oder Präkatalysatoren für das erfindungsgemäße Verfahren geeignet.

Für das vorliegende Verfahren hat sich gezeigt, daß eine Menge von 0.0001 bis 5 mol% der katalytisch aktiven Metallverbindung (berechnet auf das Metall), bevorzugt von 0.001 - 4 mol% und besonders bevorzugt von 0.01 - 2 mol% bezogen auf den Harnstoff (III), ausreichend ist.

Bevorzugt kann der Reaktion ein Halogenidsalz als Cokatalysator zugesetzt werden. Als Halogenidsalz können z. B. Phosphoniumbromide und Phosphoniumiodide, z. B. Tetrabutylphosphoniumbromid bzw. Tetrabutylphosphoniumiodid, sowie Ammonium-, Lithium-, Natrium-, Kaliumchlorid, -bromid und -iodid verwendet werden. Bevorzugte Halogenide sind die Chloride und Bromide. Vorzugsweise wird das ionische Halogenid in einer Menge von 1 bis 100 mol%, insbesondere von 2 - 40 mol% und ganz besonders von 5 - 30 mol%, bezogen auf den Harnstoff (III), eingesetzt.

Als Lösungsmittel können Wasser sowie prinzipiell alle dem Fachmann geläufigen organischen Verbindungen eingesetzt werden. Bevorzugt sind dipolar aprotische Verbindungen einsetzbar. Beispiele dafür sind Dioxan, Tetrahydrofuran, N-Methylpyrrolidon, Ethylenglykoldimethylether, Essigsäureethylester, Essigsäure, Acetonitril, Benzonitril, tert.Butylmethylether, Dibutylether, Sulfolan, DMSO, N,N-Dimethylacetamid oder Gemische davon. Die Lösungsmittel können in reiner Form oder produktenthaltend bzw. mit Produkt gesättigt eingesetzt werden. Besonders bevorzugt sind N-Methylpyrrolidon, Dimethylformamid und Acetonitril als Lösungsmittel.

Die Reaktion kann bei Drücken von 1 bis 250 bar, vorzugsweise von 10 bis 150 bar durchgeführt werden.

Bezüglich der Temperatur ist das erfindungsgemäße Verfahren relativ unkritisch. Die Reaktion kann bei Temperaturen von 0 - 200 °C, vorzugsweise von 50 - 150 °C, durchgeführt werden.

Der Aldehyd (II) wird zweckmäßigerweise in einer Menge von 0.5 bis 5 Equivalenten, bevorzugt 0.8 bis 2 Equivalenten, bezogen auf den Harnstoff, eingesetzt.

Durch den Einsatz von chiralen enantiomerenangereicherten Liganden in Kombination mit der katalytisch aktiven Metallverbindung können mittels dieses Verfahrens auch enantiomerenangereicherte Hydantoine bzw. nach Hydrolyse enantiomerenangereicherte α-Aminosäuren erhalten werden. Als chirale Liganden kommen im Prinzip alle dem Fachmann bekannten Liganden in Frage, welche an die katalytisch aktive Metallverbindung koordinieren. Eine Übersicht der in Frage kommenden Liganden bietet I. Ojima, Catalytic Asymmetric Synthesis, VCH, New York, 1993.

Besonders bevorzugt sind dabei Liganden wie 1-Diphenylphosphino-1-phenylethan, 1-Diphenylphosphino-1-naphthylethan, DIOP, BINAP, MOP, 1-Diphenylphosphino-1-ferrocenylethan, 3,4-Carbophos, Duphos, BPPM, BPE, DIPAMP, Propraphos, Deguphos, Chiraphos, Norphos, Bichep, MCCPM, Josiphos, Bimop.

Die enzymatische oder chemische Hydrolyse der Hydantoine (I) zu den α-Aminosäuren ist z.B. in Enzyme Catalysis in Organic Synthesis, K. Drauz, H. Waldmann (Eds.), VCH-Wiley, 1995 oder Houben-Weyl, Bd. 11/2, Thieme Verlag, 1958, S. 368 ff. beschrieben.

Aldehyde reagieren mit Harnstoffen in saurem Medium leicht zu unerwünschten 2-Oxo-1,2,3,4-tetrahydropyrimidinen (Monatshefte Chem. 1965, 96, 1950-1966). Für den Fachmann ist es daher überraschend, daß mit dem beschriebenen Verfahren die Hydantoine in hoher Selektivität im Eintopfverfahren erhalten werden können. Zudem hätte man erwartetet, daß Hydantoine durch das bei der Kondensation entstandene Wasser zu entsprechenden N-Carbamoylaminosäure geöffnet werden, was jedoch nicht in dem erwarteten Maß eintritt.

Unter einem (C₁-C₁₈)-Alkylrest wird im Rahmen der Erfindung ein Rest mit 1 bis 18 gesättigten C-Atomen verstanden, der beliebige Verzweigungen aufweisen kann. Insbesondere sind unter diese Gruppe die Reste Methyl, Ethyl, n-Propyl, Isopropyl, n-Butyl, Isobutyl, sec-Butyl, tert-Butyl, Pentyl, Hexyl etc. subsumierbar. Ein (C₁-C₈)-Alkylrest beschreibt den eben definierten Rest im Umfang von 1 bis 8 C-Atomen.

Ein (C₂-C₁₈)-Alkenylrest weist die für den (C₁-C₁₈)-Alkylrest genannten Merkmale auf, wobei innerhalb des Restes mindestens eine Doppelbindung vorhanden sein muß.

Ein (C₂-C₁₈)-Alkinylrest weist die für den (C₁-C₁₈)-Alkylrest genannten Merkmale auf, wobei innerhalb des Restes mindestens eine Dreifachbindung vorhanden sein muß.

Unter einem (C₆-C₁₈)-Arylrest wird ein aromatischer Rest mit 6 bis 18 C-Atomen verstanden. Insbesondere zählen hierzu Reste wie Phenyl-, Naphthyl-, Anthryl-, Phenanthryl-, Biphenylreste.

Ein (C₇-C₁₉)-Aralkylrest ist ein über einen (C₁-C₈)-Alkylrest an das Molekül gebundener (C₆-C₁₈)-Arylrest.

Ein (C₃-C₁₈)-Heteroarylrest bezeichnet im Rahmen der Erfindung ein fünf-, sechs- oder siebengliedriges aromatisches Ringsystem aus 3 bis 18 C-Atomen, welches Heteroatome wie z.B. Stickstoff, Sauerstoff oder Schwefel im Ring aufweist. Als solche Heteroaromaten werden insbesondere Reste angesehen, wie 1-, 2-, 3-Furyl, 1-, 2-, 3-Pyrrolyl, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, Acridinyl, Chinolinyl, Phenanthridinyl, 2-, 4-, 5-, 6-Pyrimidinyl.

Unter einem (C₄-C₁₉)-Heteroaralkyl wird ein dem (C₇-C₁₉)-Aralkylrest entsprechendes heteroaromtisches System verstanden.

Mithin bezeichnet im Rahmen der Erfindung ein (C₃-C₈)-Cycloalkylrest einen Rest der Gruppe der cyclischen Alkylreste mit 3 bis 8 C-Atomen und ggf. beliebiger Verzweigung. Insbesondere sind unter diese Gruppe die Reste Cyclopropyl, Cyclobutyl, Cyclopentyl, Cyclohexyl, Cycloheptyl zu subsumieren. In diesem Rest kann eine oder mehrere Doppelbindungen vorhanden sein.

Die eben genannten Alkyl-, Alkenyl- und Aryl-/Heteroarylgruppen können zusätzlich mit NO₂, NO, NOH, NNH₂, NN[(C₁-C₈)-Alkyl]₂, NNH-(C₁-C₈)-Alkyl, CN, CHO, NHCO-(C₁-C₁₈)-Alkyl, CO-(C₁-C₁₈)-Alkyl, NHCHO, CO-(C₁-C₁₈)-Aryl, CO₂-(C₁-C₁₈)-Aryl, CF₃, CCl₃, CON[(C₁-C₁₈)-Alkyl]₂, OCO-(C₁-C₁₈)-Alkyl, OCONH-(C₁-C₁₈)-Alkyl, OCON[(C₁-C₁₈)-Alkyl]₂, CONH-(C₆-C₁₈)-Aryl, CON[(C₆-C₁₈)-Aryl]₂, OCO-(C₆-C₁₈)-Aryl, OCONH-(C₆-C₁₈)-Aryl, OCON[(C₆-C₁₈)-Aryl]₂, CHCHCO₂-(C₁-C₁₂)-Alkyl, Si[(C₁-C₁₈)-Alkyl]₃, Si[O-(C₁-C₁₈)-Alkyl]₃, Si Si[NH-(C₁-C₁₈)-Alkyl]₃ substituiert sein.

Unter Hal versteht man Fluor, Chlor, Brom ,Iod.

Unter dem Begriff enantiomerenangereichert wird im Rahmen der Erfindung der Anteil eines Enantiomers im Gemisch mit seiner optischen Antipode in einem Bereich von >50% und <100% verstanden.

Unter dem Substituenten PO₀₋₃R¹R² sind alle dieser Summenformel entsprechenden Reste zu verstehen, ungeachtet dessen, daß die Reste R¹ und R² direkt oder über einen Sauerstoff an das Phosphoratom gebunden sind.

Die folgenden Beispiele sollen das Verfahren erläutern.

### Beispiel 1:

2.800 g Cyclohexancarbaldehyd, 1.500 g Harnstoff , 25 ml N-Methylpyrrolidon, 0,017 g Palladium(II)-bromid, 0,033 g Triphenylphosphan, 0,100 g Schwefelsäure und 0,760 g Lithiumbromid werden in einem 300-ml-Autoklaven mit 60 bar bei 100°C umgesetzt. Nach einer Reaktionszeit von 12 Stunden wird das Lösungsmittel im Vakuum entfernt und der Rückstand mittels Hochdruckflüssigkeitschromatographie (HPLC) analysiert. Man findet 3.200 g 5-Cyclohexyl-hydantoin. Das entspricht einer Ausbeute von 70%.

### Beispiel 2:

2.800 g Cyclohexancarbaldehyd, 1.900 g N-Methylharnstoff , 25 ml N-Methylpyrrolidon, 0,017 g Palladium(II)-bromid, 0,033 g Triphenylphosphan, 0,100 g Schwefelsäure und 0,760 g Lithiumbromid werden in einem 300-ml-Autoklaven mit 60 bar bei 80°C umgesetzt. Nach einer Reaktionszeit von 12 Stunden wird das Lösungsmittel im Vakuum entfernt und der Rückstand mittels Hochdruckflüssigkeitschromatographie (HPLC) analysiert. Man findet 4.200 g 5-Cyclohexyl-3-methylhydantoin. Das entspricht einer Ausbeute von 86%.

### Beispiel 3:

2.600 g Benzaldehyd, 1.500 g Harnstoff, 25 ml N-Methylpyrrolidon, 0,017 g Palladium(II)-bromid, 0,033 g Triphenylphosphan, 0,100 g Schwefelsäure und 0,760 g Lithiumbromid werden in einem 300-ml-Autoklaven mit 60 bar bei 80°C umgesetzt. Nach einer Reaktionszeit von 12 Stunden wird das Lösungsmittel im Vakuum entfernt und der Rückstand mittels Hochdruckflüssigkeitschromatographie (HPLC) analysiert. Man findet 2.200 g 5-Phenylhydantoin. Das entspricht einer Ausbeute von 50%.

### Beispiel 4 - 10:

Analog zu Beispiel 1 wurden folgende Hydantoine dargestellt (Tabelle 1):

| Beispiel | Temperatur [°C] | R³ | R² | R¹ | Ausbeute [%] |
|---|---|---|---|---|---|
| 4 | 80 | H | Me | Ph | 75 |
| 5 | 100 | Me | Me | Cyclohexyl | 79 |
| 6 | 100 | Me | Me | Ph | 85 |
| 7 | 120 | Me | Me | Isobutyl | 55 |
| 8 | 100 | H | Ph | Cyclohexyl | 64 |
| 9 | 100 | H | Bz | Ph | 50 |
| 10 | 100 | H | Et | Cyclohexyl | 51 |

### Beispiel 11: Aktivkohle

Die Reaktion wurde analog Beispiel 1 durchgeführt, als Katalysator wurde jedoch 1 mol% Pd auf Aktivkohle verwendet. Man erhält 4.300 g
5-Cyclohexyl-3-methylhydantoin. Das entspricht einer Ausbeute von 88 %.

## Patentansprüche

1. Verfahren zur Herstellung von Verbindungen der allgemeinen Formel (I) 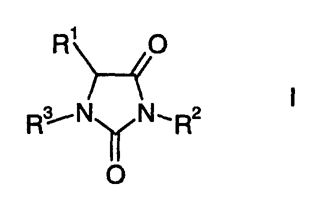 worin
R¹ bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl-(C₁-C₈)-Alkyl,
wobei die oben genannten Reste einfach oder mehrfach mit Hal, NR¹R², PO₀₋₃R¹R², OPO₀₋₃R¹R², OR¹, SR¹, SOR¹, SO₂R¹, SO₃R¹, CO₂H, CO₂R¹, CONH₂, CONHR¹ bzw. eine oder mehrere CH₂-Gruppen durch NR¹, PR¹, O oder S substituiert sein können,
R² bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈)-Cycloalkyl- (C₁-C₈)-Alkyl,
wobei die oben genannten Reste einfach oder mehrfach mit Hal, NR¹ R², PO₀₋₃R¹R², OPO₀₋₃R¹R², OR¹, SR¹, SOR¹, SO₂R¹, SO₃R¹ substituiert sein können bzw. eine oder mehrere CH₂-Gruppen durch NR¹, PR¹, O oder S substituiert sein können,
R³ bedeutet H, (C₁-C₁₈)-Alkyl, (C₂-C₁₈)-Alkenyl, (C₂-C₁₈)-Alkinyl, (C₆-C₁₈)-Aryl, (C₇-C₁₉)-Aralkyl, (C₃-C₁₈)-Heteroaryl, (C₄-C₁₉)-Heteroaralkyl, (C₁-C₈)-Alkyl-(C₆-C₁₈)-Aryl, (C₁-C₈)-Alkyl-(C₃-C₁₉)-Heteroalkyl, (C₃-C₈)-Cycloalkyl, (C₁-C₈)-Alkyl-(C₃-C₈)-Cycloalkyl, (C₃-C₈) -Cycloalkyl- (C₁-C₈)-Alkyl,
wobei die oben genannten Reste einfach oder mehrfach mit Hal, NR¹R², PO₀₋₃R¹R², OPO₀₋₃R¹R², OR¹, SR¹, SOR¹, SO₂R¹, SO₃R¹ substituiert sein können bzw. eine oder mehrere CH₂-Gruppen durch NR¹, PR¹, O oder S substituiert sein können,
**dadurch gekennzeichnet, daß** man
eine Verbindung der allgemeinen Formel (II) 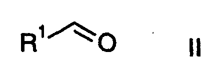 worin R¹ die oben angegebene Bedeutung annehmen kann, mit einer Verbindung der allgemeinen Formel (III) 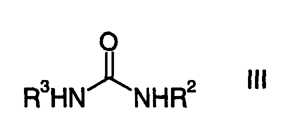 worin R² und R³ die oben angegebenen Bedeutungen annehmen können, in Gegenwart von CO und einer Pd-(0)-Verbindung oder Co-(0)-Verbindung oder Ir-(+1)-Verbindung oder Ir-(+3)-Verbindung oder Mn-(0)-, Mn-(+2)-, Mn-(+3)-Verbindung umsetzt.

2. Verfahren nach Anspruch 1,
**dadurch gekennzeichnet, daß**
R¹ einen Rest ausgewählt aus der Gruppe Methyl, Ethyl, Isopropyl, n-Butyl, sec-Butyl, Isobutyl, tert-Butyl, Methylthioethyl, Thiomethyl, Methoxycarbonylmethyl, Methoxycarbonylethyl, Phenyl, 2-, 3-, 4-Pyridyl, Benzyl, 1-, 2-Phenylethyl, Hydroxymethyl, Hydroxyethyl, Vinyl, Methoxymethyl, Methoxyethyl, Carboxymethyl, Carboxyethyl, Acetamidomethyl, Acetamidoethyl, Chlormethyl, Chlorethyl, Methylphosphonoethyl, 2-Ethylhexyl, Tetradecyl, Hexadecyl bedeutet und R² und R³ unabhängig voneinander Wasserstoff, Methyl, Ethyl, Butyl, Phenyl, Benzyl, 2-Ethylhexyl bedeuten können, R² und R³ unabhängig voneinander Wasserstoff, Methyl, Ethyl, Butyl, Phenyl, Benzyl, 2-Ethylhexyl bedeuten können.

3. Verfahren nach Anspruch 1 oder 2,
**dadurch gekennzeichnet, daß** man
die Umsetzung in Gegenwart einer Säure mit einem pKs-Wert von < 5 durchführt.

4. Verfahren nach einem oder mehreren der Ansprüche 1 bis 3,
**dadurch gekennzeichnet, daß** man
die Umsetzung in Gegenwart von Schwefelsäure oder einem Hydrogenhalogenid durchführt.

5. Verfahren nach einem oder mehreren der Ansprüche 1 bis 4,
**dadurch gekennzeichnet, daß** man
den Katalysator in einer Menge von 0,0001 bis 5 mol%, bezogen auf den Harnstoff einsetzt.

6. Verfahren nach einem oder mehreren der Ansprüche 1 bis 5,
**dadurch gekennzeichnet, daß** man
der Reaktion ein Halogenidsalz in einer Konzentration von 0.1 bis 100 mol% bezogen auf den Harnstoff zusetzt.

7. Verfahren nach einem oder mehreren der Ansprüche 1 bis 6,
**dadurch gekennzeichnet, daß** man
als Lösungsmittel N-Methylpyrrolidin, Dimethylformamid oder Acetonitril einsetzt.

8. Verfahren nach einem oder mehreren der Ansprüche 1 bis 7,
**dadurch gekennzeichnet, daß** man
bei einem CO-Druck von 1-250 bar, arbeitet.

9. Verfahren nach einem oder mehreren der Ansprüche 1 bis 8,
**dadurch gekennzeichnet, daß** man
bei einer Temperatur von 0 bis 200°C, arbeitet.

10. Verfahren nach einem oder mehreren der Ansprüche 1 bis 9,
**dadurch gekennzeichnet, daß** man
den Aldehyd in einer Menge von 0.5 bis 5 Equivalenten, bei der Reaktion einsetzt.

11. Verfahren nach einem oder mehreren der Ansprüche 1 bis 10,
**dadurch gekennzeichnet, daß** man
zur Herstellung der enantiomerenangereicherten Verbindungen der allgemeinen Formel (I) chiral modifizierte Metallkatalysatoren verwendet.

## Claims

1. Process for the preparation of compounds of the general formula (I) 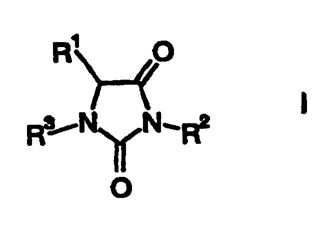 wherein
R¹ denotes H, (C₁-C₁₈)-alkyl, (C₂-C₁₈)-alkenyl, (C₂-C₁₈)-alkinyl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl-(C₆-C₁₈)-aryl, (C₁-C₈)-alkyl-(C₃-C₁₉)-heteroalkyl, (C₃-C₈)-cycloalkyl, (C₁-C₈)-alkyl- (C₃-C₈)-cycloalkyl, (C₃-C₈)-cycloalkyl-(C₁-C₈)-alkyl,
where the abovementioned radicals can be mono- or polysubstituted by Hal, NR¹R², PO₀₋₃R¹R², OPO₀₋₃R¹R², OR¹, SR¹, SOR¹, SO₂R¹, SO₃R¹, CO₂H, CO₂R¹, CONH₂, CONHR¹ or one or more CH₂ groups can be substituted by NR¹, PR¹, O or S,
R² denotes H, (C₁-C₁₈)-alkyl, (C₂-C₁₈)-alkenyl, (C₂-C₁₈)-alkinyl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl-(C₆-C₁₈)-aryl, (C₁-C₈)-alkyl-(C₃-C₁₉)-heteroalkyl, (C₃-C₈)-cycloalkyl, (C₁-C₈)-alkyl-(C₃-C₈)-cycloalkyl, (C₃-C₈)-cycloalkyl-(C₁-C₈)-alkyl,
where the abovementioned radicals can be mono- or polysubstituted by Hal, NR¹ R², PO₀₋₃R¹R², OPO₀₋₃R¹R², OR¹, SR¹, SOR¹, SO₂R¹, SO₃R¹ or one or more CH₂ groups can be substituted by NR¹, PR¹, O or S.
R³ denotes H, (C₁-C₁₈)-alkyl, (C₂-C₁₈)-alkenyl, (C₂-C₁₈)-alkinyl, (C₆-C₁₈)-aryl, (C₇-C₁₉)-aralkyl, (C₃-C₁₈)-heteroaryl, (C₄-C₁₉)-heteroaralkyl, (C₁-C₈)-alkyl-(C₆-C₁₈)-aryl, (C₁-C₈)-alkyl-(C₃-C₁₉)-heteroalkyl, (C₃-C₈)-cycloalkyl, (C₁-C₈)-alkyl-(C₃-C₈)-cycloalkyl, (C₃-C₈)-cycloalkyl-(C₁-C₈)-alkyl,
where the abovementioned radicals can be mono- or polysubstituted by Hal, NR¹R², PO₀₋₃R¹R², OPO₀₋₃R¹R², OR¹, SR¹, SOR¹, SO₂R¹, SO₃R¹ or one or more CH₂ groups can be substituted by NR¹, PR¹, O or S,
**characterized in that**
a compound of the general formula (II) 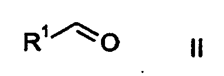 wherein R¹ can assume the abovementioned meaning, is reacted with a compound of the general formula (III) 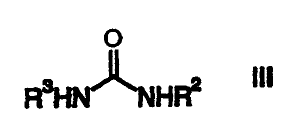 wherein R² and R³ can assume the abovementioned meanings, in the presence of CO and a Pd-(0) compound or Co-(0) compound or Ir-(+1) compound or Ir-(+3) compound or Mn-(0), Mn-(+2), Mn-(+3) compound.

2. Process according to claim 1,
**characterized in that**
R¹ denotes a radical chosen from the group consisting of methyl, ethyl, isopropyl, n-butyl, sec-butyl, isobutyl, tert-butyl, methylthioethyl, thiomethyl, methoxycarbonylmethyl, methoxycarbonylethyl, phenyl, 2-, 3-, 4-pyridyl, benzyl, 1-,2-phenylethyl, hydroxymethyl, hydroxyethyl, vinyl, methoxymethyl, methoxyethyl, carboxymethyl, carboxyethyl, acetamidomethyl, acetamidoethyl, chloromethyl, chloroethyl, methylphosphonoethyl, 2-ethylhexyl, tetradecyl, hexadecyl and R² and R³ independently of one another can denote hydrogen, methyl, ethyl, butyl, phenyl, benzyl, 2-ethylhexyl,
R² and R³ independently of one another can denote hydrogen, methyl, ethyl, butyl, phenyl, benzyl, 2-ethylhexyl.

3. Process according to claim 1 or 2,
**characterized in that**
the reaction is carried out in the presence of an acid with a pKa value of < 5.

4. Process according to one or more of claims 1 to 3,
**characterized in that**
the reaction is carried out in the presence of sulfuric acid or a hydrogen halide.

5. Process according to one or more of claims 1 to 4,
**characterized in that**
the catalyst is employed in an amount of 0.0001 to 5 mol%, based on the urea.

6. Process according to one or more of claims 1 to 5,
**characterized in that**
a halide salt is added to the reaction in a concentration of 0.1 to 100 mol%, based on the urea.

7. Process according to one or more of claims 1 to 6,
**characterized in that**
N-methylpyrrolidone, dimethylformamide or acetonitrile is employed as the solvent.

8. Process according to one or more of claims 1 to 7,
**characterized in that**
the reaction is carried out under a CO pressure of 1-250 bar.

9. Process according to one or more of claims 1 to 8,
**characterized in that**
the reaction is carried out at a temperature of 0 to 200°C.

10. Process according to one or more of claims 1 to 9,
**characterized in that**
the aldehyde is employed in the reaction in an amount of 0.5 to 5 equivalents.

11. Process according to one or more of claims 1 to 10,
**characterized in that**
to prepare the enantiomerically concentrated compounds of the general formula (I), chirally modified metal catalysts are used.

## Revendications

1. Procédé pour la préparation de composés de formule générale (I) 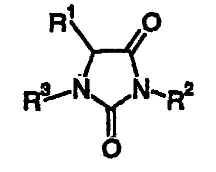 dans laquelle
R¹ représente H, un groupe alkyle en C₁-C₁₈, alcényle en C₂-C₁₈, alcynyle en C₂-C₁₈, aryle en C₆-C₁₈, aralkyle en C₇-C₁₉, hétéroaryle en C₃-C₁₈, hétéroaralkyle en C₄-C₁₉, alkyl(C₁-C₈)-aryle(C₆-C₁₈), alkyl(C₁-C₈)-hétéroalkyle(C₃-C₁₉), cycloalkyle en C₃-C₈, alkyl(C₁-C₈)-cycloalkyle(C₃-C₈), cycloalkyl(C₃-C₈)-alkyle(C₁-C₈),
les radicaux mentionnés ci-dessus pouvant être une ou plusieurs fois substitués par des atomes d'halogène ou par des groupes NR¹R², PO₀₋₃R¹R², OPO₀₋₃R¹R², OR¹, SR¹, SOR¹, SO₂R¹, SO₃R¹, CO₂H, CO₂R¹, CONH₂, CONHR¹, ou un ou plusieurs groupes CH₂ pouvant être remplacés par NR¹, PR¹, O ou S ;
R² représente H ou un groupe alkyle en C₁-C₁₈, alcényle en C₂-C₁₈, alcynyle en C₂-C₁₈, aryle en C₆-C₁₈, aralkyle en C₇-C₁₉, hétéroaryle en C₃-C₁₈, hétéroaralkyle en C₄-C₁₉, alkyl(C₁-C₈)-aryle(C₆-C₁₈), alkyl(C₁-C₈)-hétéroalkyle(C₃-C₁₉), cycloalkyle en C₃-C₈, alkyl(C₁-C₈)-cycloalkyle(C₃-C₈), cycloalkyl(C₃-C₈)-alkyle(C₁-C₈),
les radicaux mentionnés ci-dessus pouvant être une ou plusieurs fois substitués par des atomes d'halogène ou par des groupes NR¹R², PO₀₋₃R¹R², OPO₀₋₃R¹R², OR¹, SR¹, SOR¹, SO₂R¹, SO₃R¹ ou un ou plusieurs groupes CH₂ pouvant être remplacés par NR¹, PR¹, O ou S ;
R³ représente H ou un groupe alkyle en C₁-C₁₈, alcényle en C₂-C₁₈, alcynyle en C₂-C₁₈, aryle en C₆-C₁₈, aralkyle en C₇-C₁₉, hétéroaryle en C₃-C₁₈, hétéroaralkyle en C₄-C₁₉, alkyl(C₁-C₈)-aryle(C₆-C₁₈), alkyl(C₁-C₈)-hétéroalkyle(C₃-C₁₉), cycloalkyle en C₃-C₈, alkyl(C₁-C₈)-cycloalkyle(C₃-C₈), cycloalkyl(C₃-C₈)-alkyle(C₁-C₈),
les radicaux mentionnés ci-dessus pouvant être une ou plusieurs fois substitués par des atomes d'halogène ou par des groupes NR¹R², PO₀₋₃R¹R², OPO₀₋₃R¹R², OR¹, SR¹, SOR¹, SO₂R¹, SO₃R¹ ou un ou plusieurs groupes CH₂ pouvant être remplacés par NR¹, PR¹, O ou S,
en faisant réagir un composé de formule générale (II) 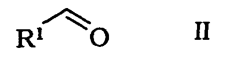 dans laquelle R¹ peut avoir la signification indiquée plus haut, avec un composé de formule générale (III) 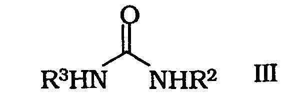 dans laquelle R² et R³ peuvent avoir les significations indiquées plus haut, en présence de CO et d'un composé à base de Pd-(O) ou d'un composé à base de Co-(0) ou d'un composé à base d'Ir-(+1) ou d'un composé à base d'Ir-(+3) ou d'un composé à base de Mn-(0), Mn-(+2), Mn-(+3).

2. Procédé selon la revendication 1,
**caractérisé en ce que**
R¹ représente un radical choisi dans l'ensemble constitué par les groupes méthyle, éthyle, isopropyle, n-butyle, sec-butyle, isobutyle, tert-butyle, méthylthioéthyle, thiométhyle, méthoxycarbonylméthyle, méthoxycarbonyléthyle, phényle, 2-, 3-, 4-pyridyle, benzyle, 1-, 2-phényléthyle, hydroxyméthyle, hydroxyéthyle, vinyle, méthoxyméthyle, méthoxyéthyle, carboxyméthyle, carboxyéthyle, acétamidométhyle, acétamidoéthyle, chlorométhyle, chloréthyle, méthylphosphonoéthyle, 2-éthylhexyle, tétradécyle, hexadécyle, et
R² et R³ peuvent représenter, indépendamment l'un de l'autre, un atome d'hydrogène ou un groupe méthyle, éthyle, butyle, phényle, benzyle, 2-éthylhexyle.

3. Procédé selon la revendication 1 ou 2,
**caractérisé en ce qu'**
on effectue la réaction en présence d'un acide ayant un pKa de < 5.

4. Procédé selon une ou plusieurs des revendications 1 à 3,
**caractérisé en ce qu'**
on effectue la réaction en présence d'acide sulfurique ou d'un halogénure d'hydrogène.

5. Procédé selon une ou plusieurs des revendications 1 à 4,
**caractérisé en ce qu'**
on utilise le catalyseur en une quantité de 0,0001 à 5 % en moles, par rapport à l'urée.

6. Procédé selon une ou plusieurs des revendications 1 à 5,
**caractérisé en ce qu'**
on ajoute au mélange réactionnel un halogénure à une concentration de 0,1 à 100 % en moles, par rapport à l'urée.

7. Procédé selon une ou plusieurs des revendications 1 à 6,
**caractérisé en ce qu'**
on utilise comme solvant la N-méthylpyrrolidine, le diméthylformamide ou l'acétonitrile.

8. Procédé selon une ou plusieurs des revendications 1 à 7,
**caractérisé en ce qu'**
on opère sous une pression de CO de 1-250 bars.

9. Procédé selon une ou plusieurs des revendications 1 à 8,
**caractérisé en ce qu'**
on opère à une température de 0 à 200°C.

10. Procédé selon une ou plusieurs des revendications 1 à 9,
**caractérisé en ce qu'**
on utilise dans la réaction l'aldéhyde en une quantité de 0,5 à 5 équivalents.

11. Procédé selon une ou plusieurs des revendications 1 à 10,
**caractérisé en ce que**,
pour la préparation des composés de formule générale (I) enrichis en énantiomères, on utilise des catalyseurs métalliques modifiés chiraux.
